# EUROPÄISCHE PATENTSCHRIFT

(11) **EP 1 563 870 B1**
(45) Veröffentlichungstag und Bekanntmachung des Hinweises auf die Patenterteilung: **05.03.2008**
(21) Anmeldenummer: 05010748.1
(22) Anmeldetag: 13.09.2002
(51) Int. Cl.: A61N 2/02

(54) **Verfahren zur Planung der Stimulation hyper/hypometabolischer Kortexareale**
Method for planning the stimulation of hyper/hypometabolic cortex areas
Méthode de planification de la stimulation des zones hyper/hypométaboliques du cortex

(43) Veröffentlichungstag der Anmeldung: 17.08.2005
(62) Teilanmeldung aus: 02020517.5
(73) Patentinhaber: BrainLAB AG, 85551 Kirchheim/Heimstetten (DE)
(72) Erfinder: Hartlep, Andreas, Dr., 83629 Naring (DE); Tanner, Philipp, Dr., 80769 München (DE); Eichhammer, Peter, Dr., 93133 Burglengenfeld (DE); Langguth, Berthold, Dr., 93049 Regensburg (DE)
(74) Vertreter: Schwabe - Sandmair - Marx

(56) Entgegenhaltungen:
- US-A- 5 644 234
- US-A1- 2002 087 062
- US-B1- 6 425 852
- ETTINGER G J ET AL: "Non-invasive functional brain mapping using registered transcranial magnetic stimulation" PROCEEDINGS OF THE IEEE WORKSHOP ON MATHEMATICAL METHODS IN BIOMEDICAL IMAGE ANALYSIS, XX, XX, 21. Juni 1996 (1996-06-21), Seiten 32-41, XP002202005
- HERWIG U ET AL: "NEURONAVIGATION IN DER PSYCHIATRIE - FOKUSSIERTE TRANSKRANIELLE MAGNETSTIMULATION BEI DEPRESSIVEN PATIENTEN" NERVENHEILKUNDE, SCHATTAUER, STUTTGART, DE, Bd. 7, Nr. 18, 1999, Seiten 353-357, XP008004455 ISSN: 0722-1541

## Beschreibung

Die vorliegende Erfindung betrifft die Planung der Stimulation hyper/hypometabolischer Kortexareale. Insbesondere betrifft sie die Planung der Stimulation, bevorzugt magnetischen Stimulation, hypermetabolischer Kortexareale, die der Manifestierung des systemischen Tinnitus zugeordnet sind.

Die magnetische Stimulation kortikaler Bereiche ist vor allem aus dem sogenannten "Brain-Mapping" bekannt, wobei direkt oder indirekt ein Kartierung von Hirnfunktionen erfolgt, um die Auffindung bestimmter funktioneller Hirnareale zu ermöglichen.

Aus der US 5,738,625 ist ein Verfahren bekannt, um Nervenzellen magnetisch zu stimulieren. Die US 5,644,234 beschreibt ein Kernspinresonanz(MR)-Verfahren, wobei die Position einer Mikrospule in einem Objekt ermittelt werden soll. Aus der WO 98/06342 sind ein Verfahren und eine Vorrichtung zur transcranialen magnetischen Stimulation des Gehirns bekannt, wobei ein etwa halbkugelförmiger von Spulen umwickelter Magnetkern zum Erzeugen eines Stimulationssignals verwendet wird. Bei der beschriebenen Vorrichtung und dem Verfahren soll die Sprachfunktion lokalisiert werden.

Die US 6,394,969 beschreibt ein Tinnitus-Masking und eine Surpressor-Vorrichtung zur Verwendung gepulsten Ultraschalls. Die US 6,366,813 beschreibt eine Vorrichtung und ein Verfahren für die intracraniale Stimulation zur optimalen Kontrolle neurologischer Krankheiten. Die US 6,402,678 beschreibt eine Einrichtung und ein Verfahren zur Behandlung von Migräne. Die US 6,425,852 beschreibt eine Vorrichtung und ein Verfahren zur transcranialen magnetischen Hirnstimulation, einschließlich der Behandlung von Depressionen und der Lokalisierung und Charakterisierung von Sprachschwierigkeiten.

Die US 6,258,032 beschreibt ein Verfahren zur Diagnose und Behandlung von Vasospasmus-Symptomen. Die US 6,132,361 beschreibt eine transcraniale Hirnstimulation. Die US 5,769,778 beschreibt eine magnetische, nicht-konvulsive Stimulationstherapie. Die US 5,061,234 beschreibt einen magnetischen Neural-Stimulator für die Neurophysiologie. Die DE 39 37 793 A1 beschreibt eine Vorrichtung zur induktiven Stimulation von erregbarem Gewebe. Die DE 44 08 110 A1 beschreibt ein Verfahren und eine Vorrichtung zur neuromagnetischen Stimulation. Die DE 199 14 762 A1 beschreibt eine Spulenanordnung zur transcranialen magnetischen Stimulation. Die DE 199 52 191 C1 beschreibt eine Vorrichtung zur Magnetstimulation von Neuronen und/oder Nervenfasern mit einer Spule und eine Verwendung von Mitteln zur Kühlung der Spule.

In NERVENHEILKUNDE, SCHATTAUER, STUTTGART, DE, Bd. 7, Nr. 18, 1999, Seiten 353-357, ist eine Artikel von HERWIG U ET AL. mit dem Titel **"NEURONAVIGATION IN DER PSYCHIATRIE - FOKUSSIERTE TRANSKRANIELLE** MAGNETSTIMULATION BEI DEPRESSIVEN PATIENTEN" veröffentlicht. Daraus ist ein Verfahren bekannt, bei dem hypometabole Areale individuell im PET identifiziert, die anatomische Lokalisation im MR koregistriert und die Magnetspule gezielt auf diese Areale navigiert wird. Es werden Hirnregionen in einem Bereich von ca. 2-4 ccm stimuliert.

Es ist die Aufgabe der vorliegenden Erfindung, ein Verfahren zur Planung der Stimulation hyper/hypometabolischer Kortexareale vorzuschlagen, bei dem sichergestellt ist, dass die Stimulationseinrichtung optimal positioniert wird. Insbesondere ist eine Aufgabe der vorliegenden Erfindung, ein solches Planungsverfahren bereitzustellen, das sich für die Stimulation, insbesondere die magnetische Stimulation hypermetabolischer Kortexareale eignet, die der Manifestierung des systemischen Tinnitus zugeordnet sind.

Erfindungsgemäß wird diese Aufgabe gelöst durch ein Verfahren zur Planung der Stimulation hyper/hypometabolischer Kortexareale, bei dem mittels eines bildgebenden Verfahrens Patientenanatomiedaten ermittelt werden, die Position der hyper/hypometabolischen Kortexareale in der Patientenanatomie sowie die Position einer Stimulationseinrichtung mittels eines medizinischen Navigationssystems erfasst werden, eine Simulation der Feldverteilung, so wie sie sich tatsächlich im Bereich der Gehirnstruktur ergeben wird, durchgeführt wird, die Position der hyper/hypometabolischen Kortexareale gegenüber der Position der Stimulationseinrichtung registriert bzw. referenziert wird, und bei dem auf der Basis der relativen Positionsinformatiönen die optimierte Positionierung der Stimulationseinrichtung geplant wird.

Durch die Einbindung des medizinischen Navigationssystem in die Planung der Stimulation der hyper/hypometabolischen Kortexareale wird eine optimierte Planung zur Verfügung gestellt, welche sicherstellt, dass die gewünschten kortikalen Bereiche mit hoher Treffgenauigkeit stimuliert werden, wodurch es erstmals auch möglich wird, die Stimulation solcher Kortexareale zu planen, die der Manifestierung des systemischen Tinnitus zugeordnet sind, wie dies gemäß einer bevorzugten Ausführungsform der vorliegenden Erfindung vorgesehen ist.

In speziellerer Ausgestaltung betrifft die Erfindung ein Verfahren, bei dem bei der Ermittlung der Patientenanatomiedaten einerseits funktionelle Anatomiedaten, durch funktionale Bilderfassungsverfahren ermittelt werden, insbesondere durch funktionelle Magnetresonanzerfassung und/oder Positronen-Emissions-Tomographie (PET), und andererseits strukturelle, im Navigationssystem positionell zuordnungsfähige Anatomiedaten durch konventionelle Bilderfassungsverfahren ermittelt werden, insbesondere durch Magnetresonanzerfassung bzw. Kernspintomographie, wobei dann die Navigations-Registrierung bzw. -Referenzierung für die funktionalen Anatomiedaten durch ein computerunterstütztes Matchingverfahren, insbesondere ein graphisches Matchingverfahren, mit den strukturellen Anatomiedaten erfolgt, so dass die funktionalen Anatomiedaten für die Navigation zur Verfügung stehen. Mit einer solchen Ausführungsvariante ist die Zuordnung solcher funktionellen Anatomiedaten und struktureller Anatomiedaten möglich bzw. deren Integration im Rahmen dieser Planung, um eine für den Behandlungserfolg optimale Navigation bzw. ein optimales Tracking zu gewährleisten.

Das mit der Erfindung verwendete Navigationssystem kann eines sein, welches auf der optischen Erfassung aktiv oder passiv abstrahlender Markeranordnungen basiert, die am Patienten, insbesondere an dessen Kopf und an der Stimulationseinrichtung angeordnet werden. Stattdessen oder zusätzlich hierzu kann ein Navigationssystem verwendet werden, welches auf der magnetischen bzw. induktiven Erfassung positionsgebender Einrichtungen, insbesondere Spulen bzw. Schwingkreise, basiert, die am Patienten, insbesondere an dessen Kopf und an der Stimulationseinrichtung angeordnet werden.

Die Stimulationseinrichtung kann eine Kortex-Stimulationsspule sein.

Es ist von Vorteil, die erfassten Navigationsdaten auf einer Bildausgabe zusammen mit den Planungsergebnissen auszugeben.

In weiterer vorteilhafter Ausgestaltung wird die Stimulationseinrichtung bzw. die Stimulationsspule im Rahmen der Planung kalibriert. Ferner kann mit Vorteil für die Stimulationseinrichtung bzw. die Stimulationsspule im Rahmen der Planung eine computergestützte Feldverteilungssimulation erfolgen, mit Hilfe der die Stimulationsbereiche bestimmt werden.

Die Erfindung betrifft ferner ein Programm, das, wenn es auf einem Computer läuft oder in einen Computer geladen ist, den Computer veranlasst, ein Verfahren durchzuführen, wie es oben beschrieben wurde. Außerdem betrifft die Erfindung ein Computerprogramm-Speichermedium, das ein solches Programm aufweist.

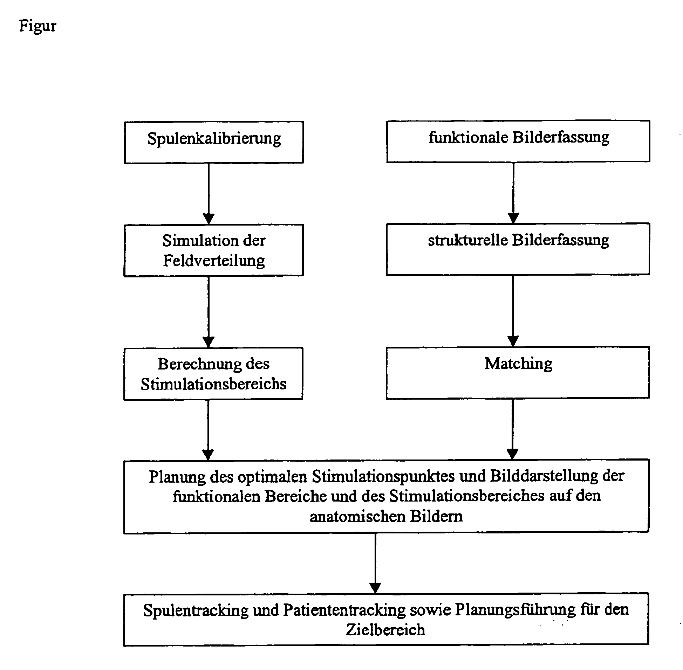

Die Erfindung wird im Weiteren anhand der beiliegenden Figur erläutert, welche als Blockdiagramm einen Ablauf des erfindungsgemäßen Planungsverfahrens aufzeigt.

Gemäß dem im Weiteren beschriebenen Ausführungsbeispiel wird die erfindungsgemäße Planung für die Tinnitus-Behandlung unter Verwendung der navigierten, transcranialen magnetischen Stimulation eingesetzt. In der ersten Phase laufen zwei Prozessteile unabhängig voneinander und möglicherweise parallel ab. Im ersten Prozessteil erfolgt die Vorbereitung der Stimulationsspule, durch die ein Magnetfeld erzeugt wird, welches bei der Behandlung die hyper/hypometabolischen Kortexareale stimulieren wird. Dabei erfolgt zunächst eine Kalibrierung der Spule und danach eine Simulation (Computersimulation) der Feldverteilung. Diese Feldverteilung wird dabei recht aufwändig schon so simuliert, wie sie sich tatsächlich im Bereich der Gehirnstruktur ergeben wird, wobei das magnetische Feld einer Spule hier natürlich sehr viel komplizierter aufgebaut ist als ohne "störende" Gehirnstruktur im Feldaufbau. Nachdem nun bekannt ist, wie die Feldverteilung aussehen wird, lässt sich auch berechnen, wo der von der Spule ausgehende Stimulationsbereich relativ zur Spulenposition sein wird. Die Spule wird, um sie positionell verfolgen zu können, mit Navigations-Markierungen (optisch, magnetisch) versehen, und sie kann damit in einem Navigationssystem getrackt werden. Dadurch können auch die vorher simulierten bzw. berechneten Simulationsbereiche getrackt werden, deren Positionsverhältnis zur Spule wie oben aufgeführt vorab ermittelt wurde.

Separat von obiger Prozessabfolge, möglicherweise zeitlich parallel, wird von einem Tinnitus-Patienten eine Bilderfassung durchgeführt. Diese Bilderfassung besteht einerseits aus der funktionalen Bilderfassung, bei der mittels funktionaler Magnetresonanzerfassung oder Positronen-Emissions-Tomographie bzw. anderer geeigneter Verfahren festgestellt wird, wo sich die funktionalen Kortexbereiche, die stimuliert werden sollen, bei diesem Patienten befinden. Im vorliegenden Fall, wo der primäre und sekundäre Hör-Kortex aufgefunden werden sollen, wird der Patient zum Beispiel Schallreizen ausgesetzt, und über die funktionale Bilderfassung wird bestimmt, welcher Kortexbereich dabei reagiert. Diese funktionalen Bilder werden also verwendet, um den Bereich der hypermetabolischen Aktivitäten im Gehirn zu bestimmen, welcher der Manifestierung des systemischen Tinnitus zugeordnet ist.

Ferner werden unabhängig hiervon Strukturbilder des Patienten erstellt, beispielsweise durch Kernspintomographie, CT oder sonstige geeignete Verfahren. Diese strukturelle Bilderfassung kann in bekannter Weise durch den Einsatz von Markern am Kopf des Patienten so in ein Navigationssystem eingeordnet werden, dass die Positionen der Gehirnstruktur gegenüber den Markern und damit im Navigationssystem verfügbar werden. Im weiteren Schritt, nämlich dem Schritt des Matching werden die erzeugten Strukturbilder gegenüber den Funktionsbildern registriert, um die räumliche Position bestimmter funktionaler Kortexareale verfügbar zu machen. Dabei kann ein graphisches, computergestütztes Matching-Verfahren verwendet werden, das die Daten der erfassten Anatomiebereiche beispielsweise anhand von in den Bildern erkennbaren Strukturabgrenzungen zuordnet.

Aus diesem Teil des Prozessablaufes erhält man demnach nach dem Matching einen Patienten-Anatomiedatensatz, mit dem bzw. auf dem navigiert werden kann und der die funktionalen Bereiche aufzeigt. Dieser Bilddatensatz wird nun verwendet, um die vorbereitete Spule zu navigieren und damit wird eine Planung des optimalen Stimulationspunktes sowie eine Bilddarstellung der funktionalen Bereiche und des Stimulationsbereiches auf den anatomischen Bildern ermöglicht. In dem kombinierten Stimulations-Neuronavigationssystem werden über eine Bilddarstellung, beispielsweise einen Bildschirm diejenigen Bereiche gezeigt, die stimuliert werden sollten, um einen Tinnitus zu behandeln. Das Navigationssystem ist dazu in der Lage, die Raumposition der Spule und des Kopfes zu erfassen und die tatsächliche und geplante Position maximaler Stimulierung zu detektieren und aufzuzeigen, um einen behandelnden Arzt zum richtigen Stimulationsgebiet (Zielbereich) hinzuführen.

## Patentansprüche

1. Verfahren zur Planung einer Stimulation hyper/hypometabolischer Kortexareale, bei dem
- mittels eines bildgebenden Verfahrens Patientenanatomiedaten ermittelt werden,
- die Position der hyper/hypometabolischen Kortexareale in der Patientenanatomie sowie die Position einer Stimulationseinrichtung mittels eines medizinischen Navigationssystems erfasst werden,
- eine Simulation der Feldverteilung, so wie sie sich tatsächlich im Bereich der Gehirnstruktur ergeben wird, durchgeführt wird,
- die Position der hyper/hypometabolischen Kortexareale gegenüber der Position der Stimulationseinrichtung registriert bzw. referenziert wird, und bei dem
- auf der Basis der relativen Positionsinformationen die optimierte Positionierung der Stimulationseinrichtung geplant wird.

2. Verfahren nach Anspruch 1, bei dem die Stimulation hypermetabolischer Kortexareale geplant wird, die der Manifestierung des systemischen Tinnitus zugeordnet sind.

3. Verfahren nach Anspruch 1 oder 2, bei dem, bei der Ermittlung der Patientenanatomiedaten einerseits funktionelle Anatomiedaten, durch funktionale Bilderfassungsverfahren ermittelt werden, insbesondere durch funktionelle Magnetresonanzerfassung und/oder Positronen-Emissions-Tomographie (PET), und andererseits strukturelle, im Navigationssystem positionell zuordnungsfähige Anatomiedaten durch konventionelle Bilderfassungsverfahren ermittelt werden, insbesondere durch Magnetresonanzerfassung bzw. Kernspintomographie, wobei dann die Navigations-Registrierung bzw. -Referenzierung für die funktionalen Anatomiedaten durch ein computerunterstütztes Matching-Verfahren, insbesondere ein graphisches Matching-Verfahren, mit den strukturellen Anatomiedaten erfolgt, so dass die funktionalen Anatomiedaten für die Navigation zur Verfügung stehen.

4. Verfahren nach einem der Ansprüche 1 bis 3, bei dem ein Navigationssystem verwendet wird, welches auf der optischen Erfassung aktiv oder passiv abstrahlender Markeranordnungen basiert, die am Patienten, insbesondere an dessen Kopf und an der Stimulationseinrichtung angeordnet worden sind.

5. Verfahren nach einem der Ansprüche 1 bis 4, bei dem ein Navigationssystem verwendet wird, welches auf der magnetischen bzw. induktiven Erfassung positionsgebender Einrichtungen, insbesondere Spulen bzw. Schwingkreise, basiert, die am Patienten, insbesondere an dessen Kopf, und an der Stimulationseinrichtung angeordnet worden sind.

6. Verfahren nach einem der Anspruche 1 bis 5, bei dem als Stimulationseinrichtung eine Kortex-Stimulationsspule verwendet wird.

7. Verfahren nach einem der Ansprüche 1 bis 6, bei dem die erfassten Navigationsdaten auf einer Bildausgabe zusammen mit den Planungsergebnissen ausgegeben werden.

8. Verfahren nach einem der Ansprüche 1 bis 7, bei dem die Stimulationseinrichtung bzw. die Stimulationsspule im Rahmen der Planung kalibriert wird.

9. Verfahren nach einem der Ansprüche 1 bis 8, bei dem für die Stimulationseinrichtung bzw. die Stimulationsspule im Rahmen der Planung eine computergestützte Feldverteilungssimulation erfolgt, mit Hilfe der die Stimulationsbereiche bestimmt werden.

10. Programm, das, wenn es auf einem Computer läuft oder in einem Computer geladen ist, den Computer veranlasst, ein Verfahren gemäß einem der Ansprüche 1 bis 9 durchzuführen.

11. Computerprogramm-Speichermedium, das ein Programm nach Anspruch 10 aufweist.

## Claims

1. A method for planning a stimulation of hyper/hypometabolic cortical areas, wherein:
- anatomical patient data are determined by means of an imaging method;
- the position of the hyper/hypometabolic cortical areas in the patient's anatomy and the position of a stimulation means are detected by means of a medical navigation system;
- the field distribution is simulated, the way it will actually be in the area of the brain structure;
- the position of the hyper/hypometabolic cortical areas is registered and/or referenced with respect to the position of the stimulation means; and wherein
- the optimal positioning for the stimulation means is planned on the basis of the relative positional information.

2. The method according to claim 1, wherein the stimulation of hypermetabolic areas, related to the manifestation of systemic tinnitus, is planned.

3. The method according to claim 1 or 2, wherein, when determining the anatomical patient data, functional anatomical data are determined on the one hand using functional image detection methods, in particular using functional magnetic resonance detection and/or positron emission tomography (PET), and on the other hand structural anatomical data which can be positionally assigned in the navigation system are determined using conventional image detection methods, in particular using magnetic resonance detection and/or nuclear spin tomography, wherein the functional anatomical data are then navigationally registered and/or referenced with the structural anatomical data using a computer-assisted matching method, in particular a graphical matching method, such that the functional anatomical data are available for navigation.

4. The method according to any one of claims 1 to 3, wherein a navigation system is used which is based on optically detecting arrangements of actively or passively emitting markers arranged on the patient, in particular on the patient's head, and on the stimulation means.

5. The method according to any one of claims 1 to 4, wherein a navigation system is used which is based on magnetically and/or inductively detecting positional means, in particular coils and/or oscillating circuits, arranged on the patient, in particular on the patient's head, and on the stimulation means.

6. The method according to any one of claims 1 to 5, wherein a cortical stimulation coil is used as the stimulation means.

7. The method according to any one of claims 1 to 6, wherein the navigational data detected are outputted, together with the planning results, on an image output.

8. The method according to any one of claims 1 to 7, wherein the stimulation means or coil is calibrated within the framework of planning.

9. The method according to any one of claims 1 to 8, wherein a computer-assisted field distribution is simulated for the stimulation means or coil within the framework of planning, with the aid of which the stimulation areas are determined.

10. A program which, when it is running on a computer or is loaded on a computer, causes the computer to perform a method in accordance with any one of claims 1 to 9.

11. A computer program storage medium comprising a program according to claim 10.

## Revendications

1. Procédé de planification d'une stimulation des zones hyper/hypométaboliques du cortex, dans lequel
- on détermine les données anatomiques du patient à l'aide d'un procédé produisant des images,
- on saisit la position des zones hyper/hypométaboliques du cortex dans l'anatomie du patient, ainsi que la position d'un dispositif de stimulation à l'aide d'un système médical de navigation,
- on réalise une simulation de la répartition du champ telle qu'elle se présente réellement au niveau de la structure cérébrale,
- on enregistre et respectivement, on référence la position des zones hyper/hypométaboliques du cortex par rapport à la position du dispositif de stimulation, et dans lequel
- on planifie le positionnement optimalisé du dispositif de stimulation sur la base des informations relatives des positions.

2. Procédé selon la revendication 1, dans lequel on planifie la stimulation des zones hypermétaboliques du cortex, qui sont rattachées à la manifestation du tinnitus systémique.

3. Procédé selon la revendication 1 ou 2, dans lequel, lors de la détermination des données anatomiques du patient, on détermine d'une part, les données fonctionnelles de l'anatomie, par un procédé fonctionnel de saisie d'images, en particulier par un examen fonctionnel de résonance magnétique et/ou tomographie à émission de positrons (PET), et d'autre part, les données structurelles de l'anatomie, susceptibles d'être rattachées par la position dans un système de navigation, par des procédés classiques de saisie d'images, en particulier par un examen de résonance magnétique et respectivement, une tomographie du spin nucléaire, où l'enregistrement et respectivement, le référencement de navigation pour les données fonctionnelles de l'anatomie, est réalisé avec les données structurelles de l'anatomie, par un procédé de correspondance assisté par ordinateur, en particulier un procédé de correspondance graphique, de sorte que les données fonctionnelles de l'anatomie sont utilisées pour la navigation.

4. Procédé selon l'une des revendications 1 à 3, dans lequel on utilise un système de navigation, qui est basé sur la saisie optique des configurations de marqueurs à rayonnement actif ou passif, qui ont été disposés sur le patient, en particulier sur sa tête et sur le dispositif de stimulation.

5. Procédé selon l'une des revendications 1 à 4, dans lequel on utilise un système de navigation, qui est basé sur la saisie magnétique et respectivement, inductive de dispositifs donnant la position, en particulier des bobines et respectivement, des circuits oscillants, qui ont été disposés sur le patient, en particulier sur sa tête et sur le dispositif de stimulation.

6. Procédé selon l'une des revendications 1 à 5, dans lequel on utilise une bobine de stimulation du cortex comme dispositif de stimulation.

7. Procédé selon l'une des revendications 1 à 6, dans lequel les données de navigation saisies sont reproduites sur un dispositif de sortie d'images avec les résultats de la planification.

8. Procédé selon l'une des revendications 1 à 7, dans lequel le dispositif de stimulation et respectivement, la bobine de stimulation est calibrée dans le cadre de la planification.

9. Procédé selon l'une des revendications 1 à 8, dans lequel on réalise une simulation de répartition de champ assistée par ordinateur pour dispositif de stimulation et respectivement pour la bobine de stimulation dans le cadre de la planification, à l'aide de laquelle on détermine les zones de stimulation.

10. Programme qui, lorsqu'il se développe sur un ordinateur ou qu'il est chargé sur un ordinateur, incite l'ordinateur à réaliser un procédé selon l'une des revendications 1 à 9.

11. Mémoire pour programme d'ordinateur, qui présente un programme selon la revendication 10.
